# EUROPEAN PATENT APPLICATION

(11) **EP 4 732 767 A1**
(43) Date of publication of application: **29.04.2026**
(21) Application number: 24208233.7
(22) Date of filing: 22.10.2024
(51) Int. Cl.: A61B 5/055, G06T 7/11, G06T 7/00

(54) **DEEP LEARNING SEQUENCE-ADAPTIVE MULTIPARAMETRIC MAGNETIC RESONANCE IMAGING QUANTIFICATION OF AN ORGAN**

(71) Applicant: Universität Bern, 3012 Bern (CH)
(72) Inventor: Huber, Adrian, 3006 Bern (CH); Zbinden, Lukas, 3600 Thun (CH)
(74) Representative: Vesterinen, Jussi Tapio

(57) **Abstract**

A computer-implemented method is proposed for quantifying one or more regions of interest (ROIs) of an organ or tumour. The method comprises: acquiring a first MRI sequence of a first sequence type, referred to a source sequence, and one or more second MRI sequences of at least a second sequence type, referred to as target sequences, the source and target sequences representing the ROIs of the organ or tumour of a patient under examination; segmenting the source sequence by using a trained AI model to obtain a labelled source sequence including a set of labels; transferring the labels from the source sequence to the target sequence(s) to obtain one or more labelled target sequences enabling identification of the ROIs in the labelled target sequence(s); and calculating ROI parametric information from voxel values identified by a respective label of the labelled target sequence(s) as processed or unprocessed to obtain region-specific information about the ROls.

## Description

### FIELD OF THE INVENTION

The present invention relates to a method for collecting region of interest (ROI) parametric information across multiparametric resonance magnetic imaging (mpMRI) volumes to obtain parametric voxel information of the ROI, where the ROI may be a portion of an organ. The method builds on a deep learning algorithm, and therefore the method provides a deep learning-based sequence-adaptive ROI quantification process. The present invention also relates a computer program product, as well as a computing apparatus configured to carry out the method.

### BACKGROUND OF THE INVENTION

Multiparametric magnetic resonance imaging (mpMRI) has emerged as a pivotal non-invasive imaging technology for detecting, characterising, and monitoring chronic liver diseases (CLD) or diseases of other organs. For the quantitative assessment of liver segmental parenchyma, mpMRI integrates various parametric sequences, including T1, T2 and R2*/T2* mapping sequences, proton density fat fraction (PDFF), diffusion-weighted intra-voxel incoherent motion (IVIM), and magnetic resonance elastography (MRE). In liver fibrosis, T1 relaxation time is increased before and decreased after contrast medium administration, while T2 relaxation time is increased in inflammation but not in fibrosis. These data may be combined with PDFF fat quantification, MRE liver stiffness measurement, and IVIM microperfusion analysis. Combination of T1 relaxation time before and after contrast medium application may be combined to obtain new parameters, such as T1 reduction rate or the extracellular volume (ECV), or with other parameters, such as PDFF (fat) and R2*/T2* (iron). Thus, mpMRI may enhance the accuracy of diagnosis and pave the way for state-of-the-art liver phenotyping, decision-making and personalised treatment planning.

Region of interest (ROI) quantification in magnetic resonance imaging (MRI) represents an important analytical technique in medical imaging, particularly in the assessment of liver diseases. This technique embodies the selective examination of specific areas or volumes within the MRI sequences, known as ROIs, to quantitatively analyse tissue characteristics. Examples of ROIs include an organ's parenchyma excluding vasculature with respect to its segmental anatomy, an organ's vasculature, such arteries and veins, and specific structures, such as cortical and subcortical structures in the brain, biliary tree in the liver or the urinary collecting system in the kidneys. ROIs may also include focal lesions, such as benign or malignant tumours. ROI quantification allows for precise measurement of parameters like tissue relaxation times, signal intensity, and textural features, which are essential for evaluating the degree of vascularization, fibrosis, stiffness, inflammation, iron or fat content. In addition, ROI may be quantified longitudinally over time to obtain information about perfusion characteristics within the ROI. By focussing on ROIs, physicians can gain a detailed understanding of localised pathological changes across time, facilitating early diagnosis, monitoring of disease progression and more informed clinical decision-making for treatment planning. Importantly, ROI quantification is also instrumental in research settings that aim to uncover correlations between MRI findings and histopathological results, or the clinical outcome under a certain therapy, thereby contributing to the progression of non-invasive diagnostic techniques and disease monitoring under treatment.

However, the detailed assessment of an organ's tissue by ROI quantification in mpMRI is a complex procedure. It requires time-consuming manual segmentation of an ROI in mpMRI sequences with dedicated software. Manual ROI quantification of mpMRI data is time-consuming, as it involves quantifying all slices within a sequence that contain the ROI, multiplied by the number of different sequences and regions of interest. To facilitate routine analysis of combined mpMRI parameters within specific ROIs of an organ or tumour, an automated analysis tool is needed. Such an approach would allow the collection of combined multiparametric information from different specific ROIs across different mpMRI sequences and/or over time, facilitating non-invasive diagnosis, clinical decision-making, and disease monitoring at significantly reduced time and cost. Beyond those obvious clinical implications, quantification of mpMRI data is instrumental in research, contributing to the development of novel non-invasive diagnostic techniques and to obtaining robust and reproducible imaging endpoints in clinical trials.

An automatic and sequence-adaptive procedure for mpMRI quantification could alleviate this manual but critical burden on physicians. Deep learning-based (DL-based) image analysis techniques may provide automated, fast, and reliable results, but they require separately trained models for each mpMRI sequence and potentially for different disease condition, limiting scalability. To our knowledge, there is currently no algorithm that integrates and combines "sequence-adaptive" analysis of previously unseen segmental mpMRI data of an organ or tumour.

### BRIEF DESCRIPTION OF THE INVENTION

The present invention aims to overcome at least some of the above-identified problems related to organ and/or tumour ROI quantifications and shall be explained in more details in liver mpMRI as an example. More specifically, when the teachings of the present invention are applied to an organ, such as the liver, we hypothesise that a procedure involving a trained algorithm for liver segmental parenchyma and liver vessels on a standard sequence, such as 3 mm non-contrast T1 volumetric interpolated breath-hold examination (VIBE) Dixon in-phase images, along with co-registration of the segmentation result to previously unseen mpMRI data using physical coordinate space affine transformations, would allow to solve this problem as explained later in more detail. The purpose of the present invention is thus to provide an automated sequence-adaptive algorithm for organ segmentation and subsequent quantification.

According to a first aspect of the invention, there is proposed a method of quantifying one or more regions of interest of an organ as recited in claim 1.

The proposed computer-implemented method is designed to automatically measure quantitative parameters of an ROI, which is a human or animal organ, or a portion thereof. Subsequently, the software may generate automated clinical reports based on those measurements by means of large vision language models (VLMs). To do so, multiparametric MRI (mpMRI) sequence data are processed by the proposed algorithm using an artificial intelligence (AI) model, which may be an artificial neural network (e.g. an nnU-Net) on a source sequence, which may be a non-contrast 3 mm T1 VIBE Dixon sequence, to generate a three-dimensional (3D) model of the organ showing its segmental regions. The 3D model can be considered as a 3D topographic map. The proposed algorithm uses this 3D topographic map to colocalise voxels of any other two-dimensional (2D), 3D, or even temporally resolved 4D sequence to the respective anatomic localisation, or tumour. Therefore, the present invention allows any MRI sequence to be processed, including new sequences that may be developed in the future, and is therefore sequence-adaptive. This allows multiple parameters to be collected and combined for one specific anatomic localisation, such as a perivascular region in one specific organ segment, tumour, or the whole organ. The generated results include parametric information, such as volumes, tissue texture information, contrast uptake over time, organ fat content in %, organ stiffness in kPa, relaxation time quantification, iron content - and many more. As the algorithm does not only detect normal organs, but also organ lesions, and post-therapeutic changes, those parameters may also be quantified for example in an organ tumour.

The final combination of the generated multiparametric information with a large VLM will optionally allow automated structured reports (ASR) to be generated. The ASR may be customised and generated in lay language for patients, in professional language for physicians, or it may be customised with multiple additional information and parameters that may be automatically filled in tables and databases for research purposes and clinical trials.

The main contribution of the present invention is that the ROI quantification is sequence-independent. A traditional approach to generate image characteristics from a sequence is to train an AI algorithm on one specific sequence at a time to generate certain specific parameters but limited by that sequence. As soon as a new sequence is developed or new parameters are needed, the algorithm must be retrained for the new sequence. The present invention uses a standard anatomic sequence to generate the 3D topographic map, which is then adapted automatically to any other present or future sequence generated during the same MRI exam. This allows, in addition, to collect and combine parameters from different sequences, such as T1 relaxation time corrected for the organ fat content, or to combine parameters at one location over time, for example before and after contrast medium administration.

According to a second aspect of the present invention, there is provided a computer program product comprising instructions for implementing the steps of the method when loaded and run on a computing apparatus.

According to a third aspect of the invention, there is proposed an apparatus for quantifying one or more regions of interest of an organ as recited in claim 15.

Other aspects of the invention are recited in the dependent claims attached hereto.

### BRIEF DESCRIPTION OF THE DRAWINGS

The invention will now be described in more detail with reference to the attached drawings, in which:
- Figure 1 schematically illustrates a system where the teachings of the present invention may be implemented;
- Figures 2a and 2b show a flowchart illustrating the DL-based sequence-adaptive ROI quantification method according to an example embodiment of the present invention;
- Figure 3 illustrates the assignment of a label from a source sequence slice to a voxel of a target sequence slice;
- Figure 4 shows example topographic maps obtained when implementing the process according to the flowcharts of Figures 2a and 2b; and
- Figure 5 shows a high-level flowchart summarising the DL-based sequence-adaptive ROI quantification method according to the example embodiment of the present invention.

### DETAILED DESCRIPTION OF THE INVENTION

It should be noted that the figures are provided merely as an aid to understanding the principles underlying the invention and should not be taken as limiting the scope of protection sought. Where the same reference numbers are used in different figures, these are intended to indicate similar or corresponding features. As utilised herein, "and/or" means any one or more of the items in the list joined by "and/or". As an example, "x and/or y" means any element of the three-element set {(x), (y), (x, y)}. In other words, "x and/or y" means "one or both of x and y." As another example, "x, y, and/or z" means any element of the seven-element set f(x), (y), (z), (x, y), (x, z), (y, z), (x, y, z)}. In other words, "x, y and/or z" means "one or more of x, y, and z." Furthermore, the term "comprise" is used herein as an open-ended term. This means that the object encompasses all the elements listed, but may also include additional, unnamed elements. Thus, the word "comprise" is interpreted by the broader meaning "include", "contain" or "comprehend". As used herein, unless otherwise specified, the use of the ordinal adjectives "first", "second", "third", etc. to describe a common object, merely indicate that different instances of like or different objects are being referred to and are not intended to imply that the objects so described must be in a given sequence, either temporally, spatially, in ranking, or in any other manner.

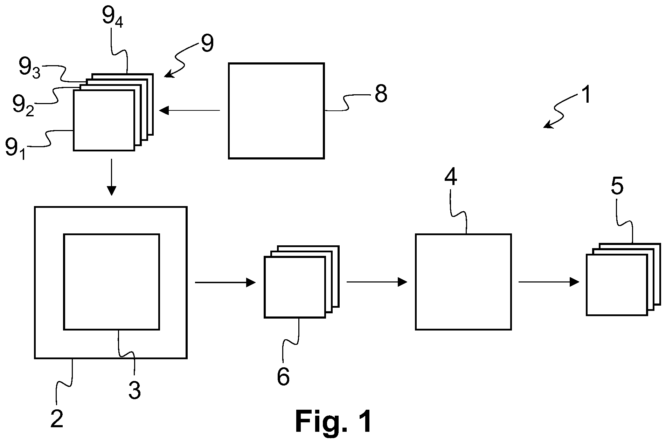

Figure 1 schematically shows an imaging or quantification system 1 where the teachings of the present invention can be applied. The system comprises a computing system 2 comprising a first AI model 3, which in this example is an artificial neural network, such as an nnU-Net. In this example, the first AI model is an nnU-Net segmentation algorithm according to the teachings of Fabian Isensee et al., "nnU-Net: a self-configuring method for deep learning-based biomedical image segmentation", Nat Methods, 7 December 2020, 18(2):203-11*.* The computing system 2 is configured to implement the DL-based sequence-adaptive ROI quantification method as explained later in more detail. The imaging system also comprises a second AI model 4 which in this example is a large VLM, and which is configured to create ASRs 5 from parametric or topographic maps 6 comprising quantified (multi)parametric information and obtained from the first computing system 2. The quantification system 1 also comprises an MRI scanner or system 8 for generating different types of MRI sequence data 9, which are arranged to be fed into the computing system 2. In this example, the mpMRI sequence data in the application phase of the process comprise a first type of MRI sequence 9₁, a second type of MRI sequence 9₂, a third type of MRI sequence 9₃, and a fourth type of MRI sequence 9₄. In the following description, the first type of MRI sequence, which may be a T1 VIBE Dixon in-phase sequence, forms a source sequence, while the second, third and fourth types of MRI sequences form target sequences, which in this example are T1 pre-contrast, T1 post-contrast, and T2, respectively. However, other types of sequences are equally possible. Thus, in the application phase, in this example, the mpMRI sequence data comprise one source sequence and a plurality of target sequences. These sequences can be visualised by three-dimensional (3D) volumes of the organ the sequences represent, where the 3D volumes are composed of voxels. It is to be noted that the teachings of the present invention are not limited to any particular number of target sequences. For example, a solution with only one target sequence is equally possible.

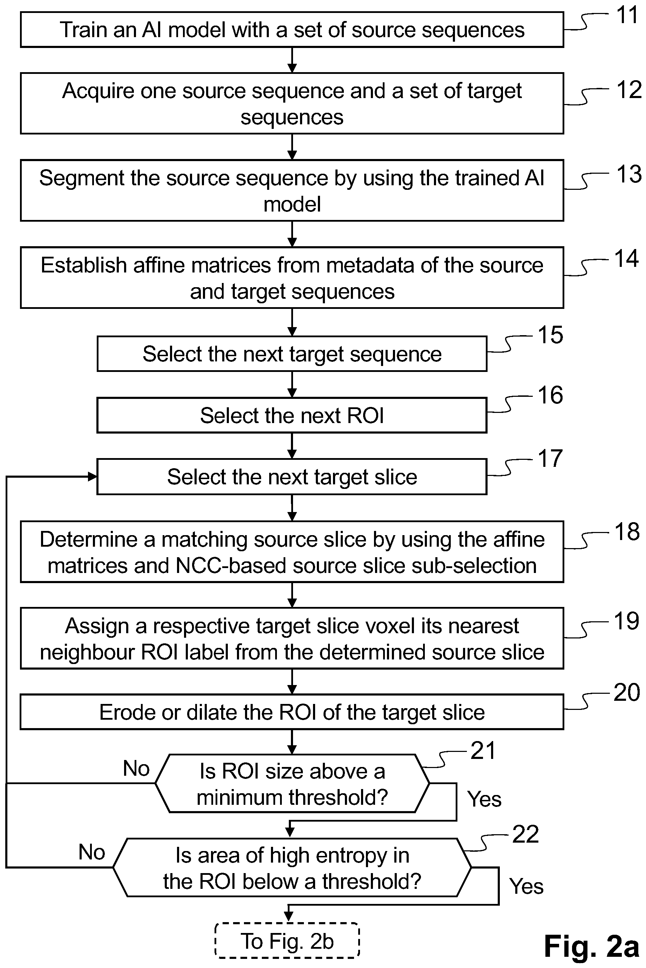

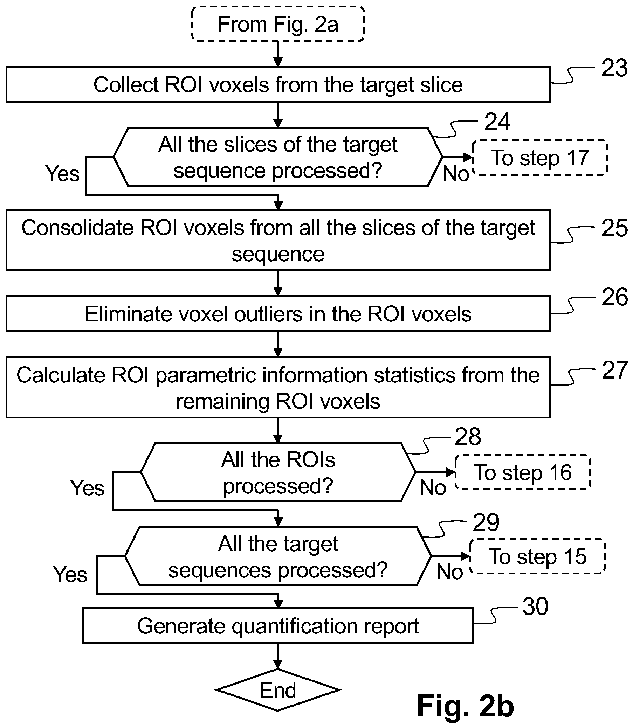

The flowcharts of Figures 2a and 2b illustrate one example embodiment of the present invention for quantifying one or more ROIs. In step 11, the first AI model 3 is trained with a set of source sequences forming a training data set. In this example, the source sequences are of the type T1 VIBE Dixon in-phase, although other source sequences, or a combination thereof, are also possible. The training data set includes source sequences obtained when performing MRI scans on a given organ, which in this example is a liver, on different patients forming a training group. Organ segmental contours and organ veins were manually labelled slice by slice to train the first AI model 3. The aim of this training step is to program the first AI model 3 so that it is able to later segment any unseen source sequence acquisition of the first type. In this example, the first AI model 3 is trained according to the teachings of Lukas Zbinden et al., "Automated liver segmental volume ratio quantification on non-contrast T1-Vibe Dixon liver MRI using deep learning", European Journal of Radiology, October 2023, Volume 167 by using the training data set to delineate liver segments and vessels according to the Couinaud classification. In this example, the task for the DL-based segmentation model is to classify each voxel into one of twelve categories, including 9 liver segments, portal vessels, hepatic vessels, and background outside of the liver.

In step 12, which starts the inference or application phase of the method, one new source sequence 9₁ (i.e. a source sequence previously unseen by the first AI model 3) and a set of target sequences 9₂, 9₃, 9₄ are acquired. In this example, three target sequences, which are of mutually different types, are acquired. These source and target sequences are generated by performing MRI scans, one MRI scan per sequence, on a patient under examination, with the requirement that all MRI sequences are collected within one MRI exam. The obtained sequences are then fed into the computing system 2. In step 13, the source sequence obtained in step 12 is segmented by using the trained first AI model 3 to obtain a segmented or labelled volume of the organ under examination, i.e., the segmented or labelled volume show different ROIs of the organ with different labels. The segmentation operation can also be called semantic segmentation, or voxel-wise classification. Volume segmentation is a process used to partition or label a volume into multiple regions or segments so that a first segment is identified with a first bit value or label, a second, different segment is identified with a second, different bit value or label, etc. making it easier to analyse and interpret specific structures within the volume. More specifically, in step 13, the 3D volume visualising the source sequence is automatically segmented by labelling each ROI with their specific or dedicated label value. In this manner, different ROIs may be visualised in the 3D volume (i.e. the topographic map) with different colours when showing the segmentation result to the user. In this specific example, the trained first AI model 3 is used for automated liver segmental labelling, excluding liver portal and hepatic veins, on data that was previously unseen by the first AI model. At the end of this step, predicted labels are obtained to thereby segment the source sequence or its visual representation.

In step 14, affine matrices are established from metadata of the source and target sequences. Affine matrices are used in linear algebra and computer graphics to represent affine transformations, which include operations such as translation, scaling, rotation, and/or shearing. An affine transformation can be described using a combination of linear transformations and translations. An affine transformation is a geometric transformation that preserves lines and parallelism, but not necessarily Euclidean distances and angles.

Next in steps 15, 16, and 17, the next target sequence, ROI, and target slice are selected by the computing system 2. Alternatively, this selection or part of it may be made by the user. More, specifically, in step 15, the next target sequence is selected, in step 16, the next ROI is selected, and as the source and target sequences or their 3D visualisations are composed of a set of source and target slices, respectively, in step 17, the next target slice is selected. The items are thus selected so that they have not yet been processed in the subsequent steps. Step 16 may also include the step of receiving a selection of target ROIs from the user if this selection has not been received earlier. Alternatively, the computing system 2 may automatically select the ROIs to be processed in the following. This automatic selection may optionally have to be approved by the user and possibly modified according to the user's preference.

In step 18, a source slice that best matches with the target slice is determined. This determination is in this example done by using the affine matrices and optionally followed by a normalised cross-correlation-based (NCC-based) source slice sub-selection. More specifically, for the current target sequence slice, the corresponding source sequence slice is determined through the affine matrices (both the source and target sequences and thus both the associated matrices are involved in this step), which gives the nearest neighbour slice in the source sequence. This can be considered a first selection. The first selection, i.e. the selected source sequence slice serves as a point of reference or starting point for an optional second selection. During the second selection, the algorithm considers the immediate neighbourhood of the selected source slice. In this manner, it may be possible to find a source slice with a higher normalised cross-correlation within the ROI as measured within the target slice. If so, then the algorithm chooses that source slice forming thus a sub-selection, otherwise the algorithm keeps the originally obtained source slice from the affine transformation.

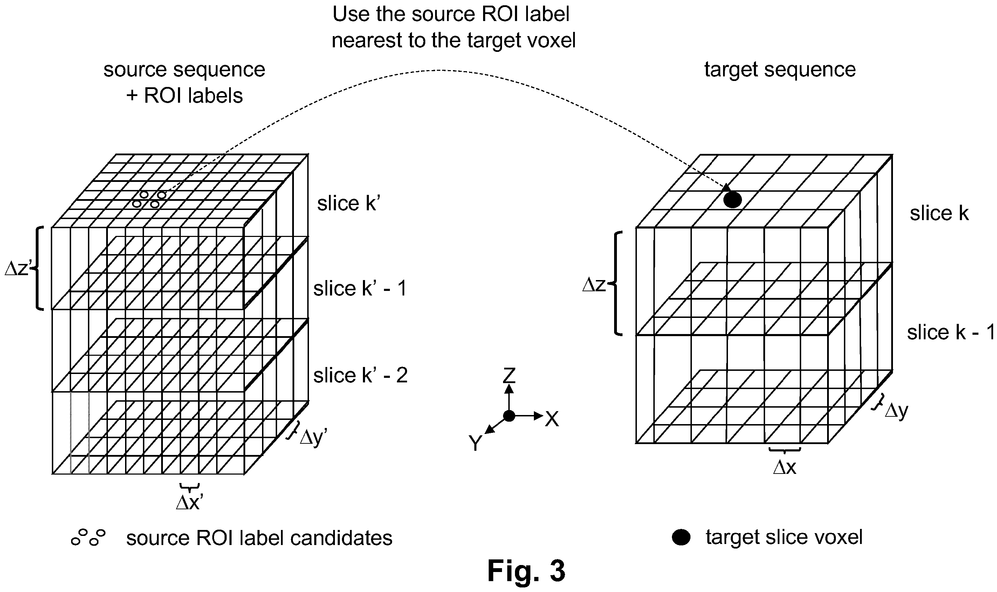

In step 19, for each target slice voxel its nearest neighbour label is identified from the determined source slice. A respective ROI is characterised or identified by its label. This step also comprises assigning the source slice labels corresponding to the ROI under examination (these labels are identified by a first label value (single value)) to the corresponding voxels in the current target slice. This is visually illustrated in Figure 3. Since the target sequence in this case has a lower resolution than the source sequence, transformation conflicts can occur where multiple source labels map to the same target voxel. These conflicts are resolved by Euclidean nearest neighbour. The voxels in the current target slice that do not spatially correspond to the current ROI are assigned another label value, i.e. a second label value (label value corresponding to the background). Thus, in the present example, the assignment operation is binary. This means that this step identifies the current ROI under examination in the target slice but no other ROIs. Once this step has been run for all the ROIs, each voxel in the target sequences has been assigned in a physical coordinate space a spatially corresponding or matching label from the source sequence so that it is possible to understand which ROI it belongs to and whether to measure it as part of a certain ROI, such as liver segment, liver vessel, or exclude it. The ultimate information that the process wants to collect therefore is the voxel itself, i.e. its intensity or parametric value, whose physical/medical interpretation depends on the specific target sequence. In other words, the algorithm is to assign or allocate each voxel in the target sequence to a predefined ROI (e.g. liver segment, liver vessel). That ROI is represented by a label which is obtained from the segmentation result of the source sequence. As explained, the first AI model 3 assigned in step 13 each voxel in the source sequence a label. Therefore, in this example, the only information that is being transferred in this step from the source sequence to the target sequence is the label holding the information which ROI a voxel belongs to. The respective target slice voxel value is maintained in the process. Ultimately and as explained later, the aim of the process is to calculate the mean voxel intensity or other parametric information of a certain ROI, such as liver segment IV. That mean voxel intensity is then interpreted depending on the specific type of target sequence, e.g. MR elastography (but there are many others), which gives a measure for tissue stiffness. Thus, by obtaining the exact delineation of a certain ROI as described above, it is possible then to precisely capture the stiffness (or another parameter value) of an ROI, such as liver segment IV. This step can thus be understood as a label mapping operation where ROI labels from the source sequence, and more precisely from the source sequence slice, are mapped to the voxels in the target sequence, and more specifically in the target sequence slice.

Optionally in step 20, the current ROI of the target slice is eroded or dilated. Erosion is a morphological operation that reduces the borders of an object or body, i.e. in this case the current ROI. In MRI, eroding the ROI along its borders aims to minimise partial volume effects. The erosion procedure is defined by a 2D kernel as the structuring element and the number of times erosion is applied. Thus, due to partial volume effects, erosion is optionally performed on the target ROI to remove potentially ambiguous voxels at the boundary. Dilation is the opposite operation whereby the size of the ROI is expanded along its borders as an "arithmetic" operation to compute more complex ROIs. For instance, to compute the perivascular ROI, i.e. the area around the liver vessels, the liver vessels are first dilated a certain amount to obtain "dilated liver vessels" ROI, then the liver vessels are subtracted from this ROI to then obtain the final perivascular ROI. Furthermore, for more sophisticated ROI calculations, erosion/dilation can be used together in a sequence (e.g., dilation followed by erosion or vice versa) for tasks like noise removal or boundary smoothing in techniques, such as opening (erosion followed by dilation) and closing (dilation followed by erosion).

In step 21, it is determined whether or not the size of the current ROI, where the size refers to the number of voxels, in the current target slice is above a minimum threshold, i.e. a minimum size threshold. If this is not the case, then the process continues in step 17, where the next slice is selected. Thus, if the size of the current ROI in terms of number of voxels is too small in the current target slice, then the current target slice is rejected, and the process moves to the next target slice. The algorithm thus optionally rejects a measurement if the size of the target ROI is below a threshold. If on the other hand the ROI is sufficiently large, then the process continues in step 22, where it is determined whether or not the area of high entropy within the ROI is below a threshold, i.e. below an entropy threshold. If this is not the case, i.e. too much high entropy occurs within the ROI, then the process again continues in step 17, where the next target slice is selected. In other words, if the amount of high entropy in the current ROI in the current target slice surpasses a threshold, then the current target slice is rejected. High entropy itself is defined by means of a threshold. In MRI axial slices, where the axial direction runs along the body and corresponds to direction Z in Figure 3, high entropy areas within the abdomen can occur due to several factors, including motion artifacts, complex anatomical structures, partial volume effects, chemical shift artifacts, and heterogeneous pathologies. Conversely, an axial or target slice of a target sequence with good acquisition quality typically exhibits comparably low entropy within the liver area. To mitigate the risk of measuring a noisy ROI, the algorithm in this example calculates the entropy within the ROI area per target slice and ensures that the fraction of high entropy values within the ROI is sufficiently small, as determined by threshold parameter *τ*. The entropy is calculated on one slice at a time (i.e. 2D only). Even though the slice has a thickness, for the entropy calculation that slice is considered as a standard matrix of (intensity) values. An entropy value is considered low if it falls below a specified threshold, referred to as parameter *threshold_{low}.* For instance, parameter *τ* may be in the range of 0.01 to 0.2, or more specifically in the range of 0.02 to 0.1, such as 0.05, while parameter *threshold_{low}* may be in the range of 1 to 3, or more specifically in the range of 1.5 to 2.5, such as 2.1, according to the entropy measure value range. The entropy itself may be calculated as base 2 logarithm local entropy with a square neighbourhood of 3 x 3 voxels. If the entropy-related constraints are not met, the algorithm omits the measurement of the ROI in the given target slice. On the other hand, if the entropy, or more specifically the size of the high entropy area compared to the total ROI area, in the current ROI in the current target slice, is below the threshold, then the process continues in step 23, where the voxels of the current ROI in the current target slice are collected.

In step 24, it is determined whether or not all the slices of the current target sequence have been processed. If this is not the case, then the process continues in step 17, where the next target sequence is selected. If on the other hand it is determined that all the slices of the current target sequence have been processed, then the process continues in step 25, where all the voxels of the current ROI from all the slices of the current target sequence are consolidated. In step 26, voxel outliers in the current ROI are eliminated. To make the ROI mean computation and the extraction of other parametric information more robust against image artifacts, noise, and outliers, statistical voxel outliers are removed. In this example, the median and the median absolute deviation (MAD) of the voxel values of the whole liver parenchyma ROI in the target sequence is calculated. The median and the MAD are then used to remove any voxels within the current ROI that are outside the range defined by median ± 3 × MAD, or more broadly median ± a deviation value. 3 × MAD as deviation value was found to work especially well, although other deviation values are also possible. In this step, the outlier voxels are thus excluded from subsequent calculations, and only the remaining voxels are later considered.

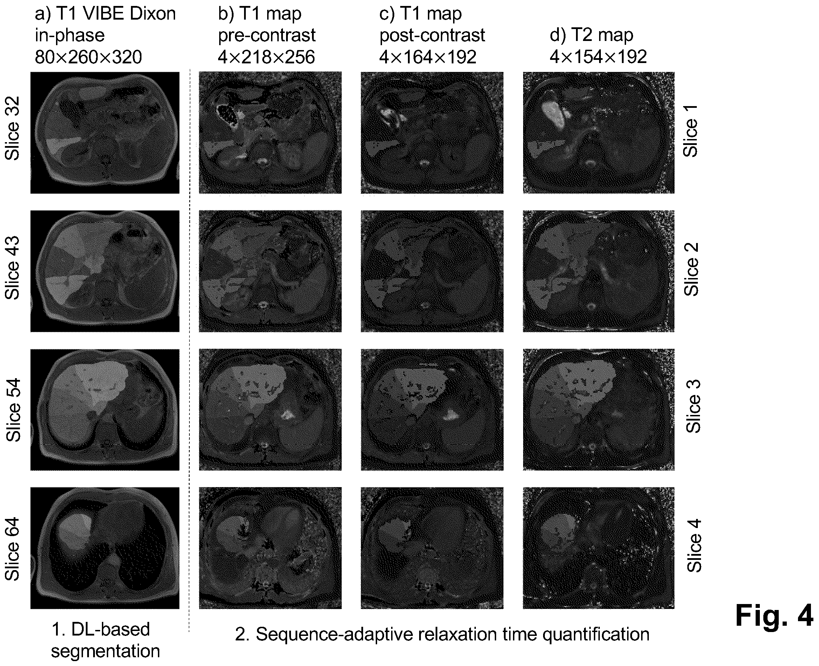

In step 27, parametric information statistics, e.g. mean, median, minimum, maximum and/or spatial distribution of voxel values, standard deviation of voxel values, number of voxels, and/or volume size of the current ROI across all the slices of the target sequence are computed from the remaining voxels in the current ROI. Preferably, all of the above metrics are computed together. Thus, the computation only considers the voxels that are left after the outlier voxels have been removed in step 26. In step 28, it is determined whether or not all the ROIs for the current target sequence have been processed. If this is not the case, the process then continues in step 16, where the next ROI is selected. If on the other hand, all the ROIs have been processed, the process then continues in step 29, where it is determined whether or not all the target sequences have been processed. If this is not the case, the process then continues in step 15, where the next target sequence is selected. If on the other hand, all the target sequences have been processed, then the process can move to step 30. Figure 4 visually illustrates the outcome of step 29 for liver segment ROIs of a single patient. More specifically, Figure 4 shows an example of automated T1 and T2 relaxation time quantification, where column a) shows four slices visualising T1 VIBE Dixon in-phase source sequence obtained by applying the pre-trained first AI model 3 to the T1 VIBE Dixon in-phase sequence, and columns b), c), and d) show how the sequence-adaptive quantification transformed the segmental labels shown in column a) to different parametric maps, including T1 map pre-contrast in column b), T1 map post-contrast in column c), T2 map in column d). In step 30, a quantification report is generated from the parametric information statistics of one or more ROIs, optionally all the ROIs, in a given target sequence. Optionally, results across at least two target sequences are combined to generate the quantification report. The quantification report is generated by the second AI model 4. After this, the process comes to an end.

It is to be noted some of the steps of the above-described process are optional and/or some of the steps may be carried out in a different order. Furthermore, it is possible to modify the process in different ways. For example, it was explained that many of the processing steps are carried out per slice. However, it would be possible to carry out these processing steps for all the slices of a given target sequence simultaneously or substantially simultaneously. Thus, instead of carrying out these processing steps per slice, they would instead be carried out per target sequence. Moreover, in the above process, it was explained that many of the processing steps are carried out per ROI. However, it would be possible to carry out these processing steps for at least two or for all the ROIs in a given slice simultaneously or substantially simultaneously. Thus, instead of carrying out these processing steps per ROI, they would instead be carried out per target slice or even per target sequence. Moreover, one or more fail-safe mechanisms may be implemented in the process. For example, if after having processed all the slices in a given ROI, it is determined that the current ROI is empty or that in step 21 the size of the ROI is below (or equal to) the threshold for all the slices, then the process can directly move to step 16, where the next ROI is selected. In a similar manner, if after having processed all the slices in a given ROI, it is determined that the current ROI is empty or that in step 22 the fraction of high entropy is never below the threshold for any of the slices in a given target sequence, then the process can directly move to step 16, where the next ROI is selected.

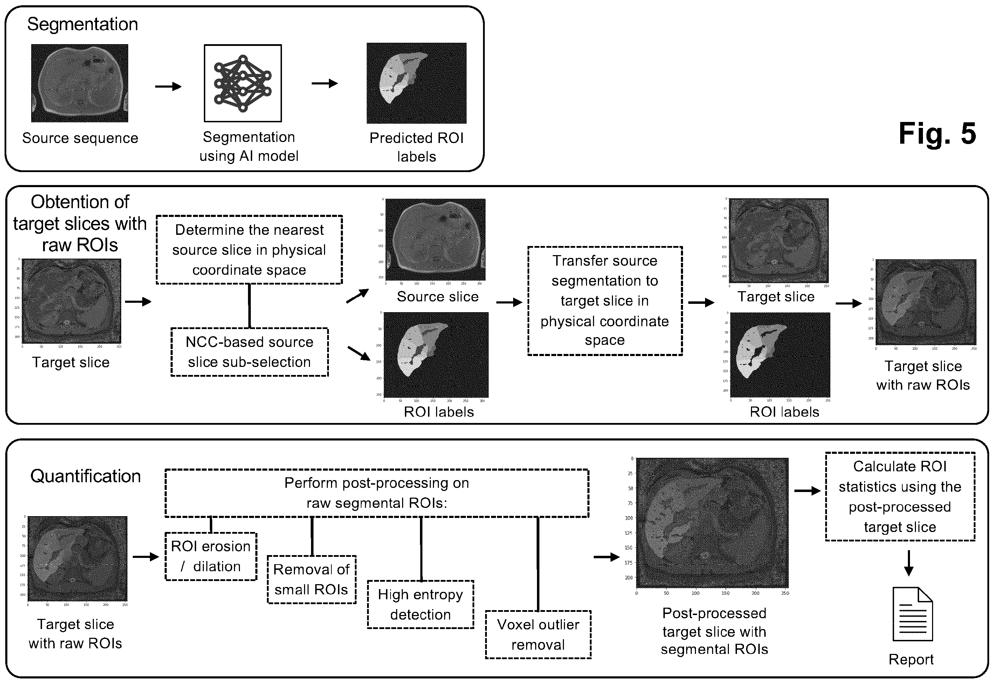

Figure 5 shows a high-level flowchart illustrating a workflow for an automated organ MRI sequence mapping quantification, which in this particular example is a workflow for automated liver MRI T1 and T2 mapping quantification. This high-level flowchart illustrates the inference or application phase of the method. This means that the first AI model 3 has already been trained by using a source sequence training dataset obtained in the training phase from a plurality of patients of the training group. As shown in Figure 5, the process can be divided into three main phases. During the first phase, the source sequence, which in this example is the non-contrast VIBE Dixon in-phase sequence, is acquired and segmented by using the trained first AI model 3 to obtain the source ROI labels. During the second phase, raw target ROIs for each target slice are obtained. More, specifically, in this example, the T1 and T2 target sequences are first acquired. Subsequently, the ROI labels from the source slices are mapped to the target slices, slice per slice and target sequence per target sequence. In other words, during the second phase, the obtained liver segmental labels (or more broadly the obtained organ regional or segmental labels) from the source sequence are co-registered to the parametric target sequences, which were all acquired simultaneously one after the other within the same MRI exam and share the same physical coordinate space, to obtain a raw segmentation of the liver segments on the parametric map, for each of the target sequences. It is to be noted that the source sequence was obtained in the same MRI session as the target sequences. At the end of this phase, target slices with raw ROIs are obtained. This means that these raw ROIs have not yet been post-processed. The design of the sequence-adaptive ROI quantification algorithm was inspired by the manual ROI quantification process. The algorithm maps the DL-based organ segmentation obtained from the source sequence to the target sequence using voxel-wise affine transformations in the physical coordinate space of the MRI scanner 8. All sequences acquired in one MRI session share the same physical coordinate space.

It is to be noted that the process according to the flowchart of Figure 5 is slightly different compared with the process explained in connection with the flowchart of Figures 2a and 2b, though the results are identical. In particular, in the example according to the flowchart of Figure 5, a respective target slice with all the raw ROIs is first obtained, and only then this respective target slice is post-processed. In the process according to the flowchart of Figures 2a and 2b, some post-processing was performed on a respective target slice as soon as a target slice with one raw ROI had been obtained. During the third phase, and as shown in Figure 5, the target slices with raw ROIs are post-processed and then ROI statistics or parametric information are calculated from the post-processed target slices when taken collectively.

As was explained above, to process the raw ROIs of the target sequences for optimal quantification accuracy, the optional post-processing steps forming additional algorithmic components were devised. Each of these components are configurable through hyperparameters. Therefore, the most effective configuration was determined through hyperparameter optimisation. Depending on the configuration, the algorithm is designed to reject the quantification of an ROI in the respective target slice, or across at least two or all target slices, if not some or all of the requirements of the components were met. In the end, the algorithm calculates the ROI mean volumetrically (3D) from all axial slices containing the same ROI.

The sequence-adaptive ROI quantification algorithm can be optimised separately from the DL-based segmentation algorithm for its hyperparameters. The algorithm requires configuration only, with no learnable parameters. For optimisation, the algorithm uses a multi-objective function that optimises two criteria simultaneously: 1) the mean intraclass correlation coefficient (ICC), as it represents a common measure in reliability and agreement analysis between a machine learning-based algorithm and an expert rater; and 2) the number of ROI measurements to ensure clinical utility. A trade-off occurs when an increased number of measurements negatively impacts the ICC value, or vice versa. ICC(1, 1) was used to compare the algorithm to the manual reference standard. Consequently, the multi-objective function can be formulated as follows: *f* = *w*₁ · *ICCₘₑₐₙ* - *w*₂ · *ICC_{std}* + *w*₃ · |*ROIs*|*ₙₒᵣₘ*, where *ICC* is the mean and standard deviation over all ROIs, |*ROIs*|*ₙₒᵣₘ* is the normalised number of measurements over all ROIs, and *w*₁, *w*₂, *w*₃ the weights for the respective criteria. The optimisation can be performed separately for each type of ROI, i.e. in the liver example for each of the nine Couinaud liver segments. The parameter search space includes all the proposed algorithm components as described above. Grid search can be applied to exhaustively search all combinations of hyperparameter values to find a Pareto optimal algorithm configuration.

While the invention has been illustrated and described in detail in the drawings and foregoing description, such illustration and description are to be considered illustrative or exemplary and not restrictive, the invention being not limited to the disclosed embodiment. Other embodiments and variants are understood and can be achieved by those skilled in the art when carrying out the claimed invention, based on a study of the drawings, the disclosure and the appended claims. New embodiments may be obtained by combining any of the teachings above.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A computer-implemented method for quantifying one or more regions of interest forming or being part of an animal or human organ and/or tumour, the method comprising:
• acquiring (12) a first magnetic resonance imaging sequence of a first sequence type, referred to a source sequence (9₁), and one or more second magnetic resonance imaging sequences of at least a second sequence type, referred to as target sequences (9₂, 9₃, 9₄), the source and target sequences (9₁, 9₂, 9₃, 9₄) representing the one or more regions of interest of the organ and/or tumour of a patient under examination;
• segmenting (13) the source sequence (9₁) by using a trained artificial intelligence model (3) to obtain a labelled source sequence including a set of labels such as a respective label identifies a respective region of interest;
• transferring (19) the labels from the source sequence (9₁) to the one or more target sequences (9₂, 9₃, 9₄) by a mapping operation so that a respective voxel in a respective target sequence (9₂, 9₃, 9₄) is assigned a spatially corresponding label from the labelled source sequence to obtain one or more labelled target sequences enabling identification of the one or more regions of interest in the one or more labelled target sequences; and
• calculating (27) region of interest parametric information from voxel values identified by a respective label of the one or more labelled target sequences as processed or unprocessed to obtain region-specific information about the one or more regions of interest.

2. The method according to claim 1, wherein the method further comprises training (11) the artificial intelligence model (3) by using magnetic resonance imaging sequence data of the first sequence type to obtain the trained artificial intelligence model (3), the magnetic resonance imaging sequence data of the first sequence type representing the one or more regions of interest of training patients.

3. The method according to claim 1 or 2, wherein the source and target sequences (9₁, 9₂, 9₃, 9₄) or their visualisations are composed of a set of source and target slices, respectively, wherein the method further comprises determining (18) for a respective target slice the nearest source slice in a physical coordinate space, and wherein the mapping is carried out slice by slice by using voxel-wise transformations between the respective mutually nearest source and target slices.

4. The method according to claim 3, wherein the method further comprises establishing (14) affine matrices from metadata of the source and target sequences (9₁, 9₂, 9₃, 9₄) and using the affine matrices to determine the nearest source slice.

5. The method according to claim 3 or 4, wherein the voxel-wise transformations are used as a first selection to determine respective candidate source slices for the nearest source slice, and wherein the method further comprises subsequently carrying out a second selection among the respective candidate source slices to determine the nearest source slice by using normalised cross-correlation-based source slice selection.

6. The method according to any one of the preceding claims, wherein the method further comprises eroding or dilating (20) a respective region of interest along its border in a respective labelled target sequence as processed or unprocessed, and wherein the erosion or dilation is optionally carried out slice by slice in the respective labelled target sequence as processed or unprocessed.

7. The method according to any one of the preceding claims, wherein the source and target sequences (9₁, 9₂, 9₃, 9₄) or their visualisations are composed of a set of source and target slices, respectively, and wherein the method further comprises determining (21) the size of a respective region of interest in a respective target slice in a respective labelled target sequence as processed or unprocessed, and rejecting (21) the respective target slice if the size of the respective region of interest is below a size threshold.

8. The method according to any one of the preceding claims, wherein the source and target sequences (9₁, 9₂, 9₃, 9₄) or their visualisations are composed of a set of source and target slices, respectively, and wherein the method further comprises determining (22) the size of the area of high entropy relative to the area of low entropy in a respective region of interest in a respective target slice in a respective labelled target sequence as processed or unprocessed, and rejecting (21) the respective target slice if the relative size of the area of high entropy is above an entropy threshold.

9. The method according to any one of the preceding claims, wherein the method further comprises eliminating (26) voxel outliers in a respective region of interest in a respective labelled target sequence as processed or unprocessed.

10. The method according to claim 9, wherein the voxel outliers are eliminated by calculating the median of voxel values of the whole organ and/or tumour in the respective target sequence as processed or unprocessed, and removing any voxels within the respective region of interest that are outside the range defined by the median ± a deviation value.

11. The method according to claim 10, wherein the deviation value equals 3 × median absolute deviation of the voxel values in the respective region of interest.

12. The method according to any one of the preceding claims, wherein the organ is a liver, and wherein the source sequence (9₁) is a T1 VIBE Dixon in-phase sequence, and/or wherein the one or more target sequences (9₂, 9₃, 9₄) have a lower resolution than the source sequence (9₁).

13. The method according to any one of the preceding claims, wherein the region of interest parametric information is at least any one of the following: mean, median, minimum, maximum, and/or spatial distribution of voxel values, standard deviation of voxel values, number of voxels, and/or volume size of a respective region of interest in the one or more labelled target sequences as processed or unprocessed, and wherein the method optionally comprises generating (30) a quantification report from the region of interest parametric information of one or more regions of interest in the one or more labelled target sequences as processed or unprocessed, optionally of all the regions of interest in a respective labelled target sequence as processed or unprocessed, and wherein optionally the region of interest parametric information across at least two labelled target sequences as processed or unprocessed are combined to generate the quantification report.

14. A computer program product comprising instructions for implementing the steps of the method according to any one of the preceding claims when loaded and run on a computing apparatus (2).

15. A computing apparatus (2) for quantifying one or more regions of interest forming or being part of an animal or human organ and/or tumour, the computing apparatus comprising means for:
• acquiring a first magnetic resonance imaging sequence of a first sequence type, referred to a source sequence (9₁), and one or more second magnetic resonance imaging sequences of at least a second sequence type, referred to as target sequences (9₂, 9₃, 9₄), the source and target sequences (9₁, 9₂, 9₃, 9₄) representing the one or more regions of interest of the organ and/or tumour of a patient under examination;
• segmenting the source sequence (9₁) by using a trained artificial intelligence model (3) to obtain a labelled source sequence including a set of labels such as a respective label identifies a respective region of interest;
• transferring the labels from the source sequence (9₁) to the one or more target sequences (9₂, 9₃, 9₄) by a mapping operation so that a respective voxel in a respective target sequence (9₂, 9₃, 9₄) is assigned a spatially corresponding label from the labelled source sequence to obtain one or more labelled target sequences enabling identification of the one or more regions of interest in the one or more labelled target sequences; and
• calculating region of interest parametric information from voxel values identified by a respective label of the one or more labelled target sequences as processed or unprocessed to obtain region-specific information about the one or more regions of interest.
